Europäisches Patentamt

(19) European Patent Office

Office européen des brevets

(11) Veröffentlichungsnummer: **0 007 041**
**B1**

(12) **EUROPÄISCHE PATENTSCHRIFT**

(45) Veröffentlichungstag der Patentschrift:
25.11.81

(51) Int. Cl.³: **C 07 C 49/643**, C 07 C 45/65,
B 01 J 23/16, B 01 J 23/70

(21) Anmeldenummer: **79102175.1**

(22) Anmeldetag: **29.06.79**

(54) Verfahren zur Herstellung von Anthron.

(30) Priorität: **11.07.78 DE 2830456**

(43) Veröffentlichungstag der Anmeldung:
**23.01.80 Patentblatt 80/2**

(45) Bekanntmachung des Hinweises auf die Patenterteilung:
**25.11.81 Patentblatt 81/47**

(84) Benannte Vertragsstaaten:
**CH DE FR GB**

(56) Entgegenhaltungen:
**Chemisches Zentralblatt, Band 97, Nr. 7, 1926/I
Berlin
J. v. BRAUN et al., »Katalytische Hydrierungen
unter Druck bei Gegenwart von Nickelsalzen. IX.
Anthrachinon, Phenanthrenchinon und Benzanthron«
* Seiten 1553 bis 1556 *
Chemisches Zentralblatt, Band 98, Nr. 8, 1927/II
Berlin**

(73) Patentinhaber: **BAYER AG, Zentralbereich Patente,
Marken und Lizenzen, D-5090 Leverkusen 1, Bayerwerk
(DE)**

(72) Erfinder: **Schroeder, Bernd, Dr., Im Wiesengrund 4,
D-5068 Odenthal (DE)**
Erfinder: **Neeff, Rütger, Dr.,
Berta-von-Suttner-Strasse 22, D-5090 Leverkusen 1 (DE)**
Erfinder: **Braden, Rudolf, Dr., Nothauser Feld 1,
D-5068 Odenthal (DE)**

**J. D. RIEDEL AG, »Herstellung von Anthranolen
(Anthronen) und deren Kernhydrierungsprodukte«
* Seite 1087 *
Beilsteins Handbuch der Organischen Chemie,
III. Ergänzungswerk, 4. Auflage, Band 7,
1968, SPRINGER VERLAG, Berlin, Heidelberg,
New York
* Seite 2360 ***

Verfahren zur Herstellung von Anthron

Die vorliegende Erfindung betrifft ein Verfahren zur Herstellung von Anthron durch katalytische Hydrierung von Anthrachinon.

In Ber., 58, 2675 (1925), ist beschreiben, daß man Anthron durch katalytische Hydrierung von Anthrachinon in Dekalin als Lösungsmittel bei 160 – 170° C unter Druck in Gegenwart von Nickel in hoher Reinheit und praktisch quantitativer Ausbeute erhält.

Wie Vergleichsversuche zeigen, entstehen jedoch mit verschiedenen Nickel-Katalysatoren höher hydrierte Produkte. Anthron tritt im Reaktionsgemisch nicht in nennenswerter Menge auf.

Die GB-A-248 759 beschreibt die katalytische Hydrierung von Anthrachinon zu Anthron in Gegenwart eines Ni-Co-Cu-Katalysators. Die DE-A-369 374 beschreibt Möglichkeiten zur Steigerung der Wirksamkeit von Katalysatorsystemen für die Hydrierung von Kohlenwasserstoffsystemen, durch den Zusatz von Schwermetallen zu Katalysatoren, die zwei Metalle der Eisengruppe enthalten.

Es wurde nun gefunden, daß man Anthron aus Anthrachinon in guter Reinheit und hoher Ausbeute erhalten kann, wenn man Anthrachinon in inerten organischen Lösungsmitteln, wie geradkettigen oder verzweigten aliphatischen oder cycloaliphatischen Kohlenwasserstoffen, Alkoholen oder Ethern, in Gegenwart von gegebenenfalls auf Trägermaterialien aufgebrachten Katalysatoren mit 10 – 60 Gew.-% Cu, 10 bis 50 Gew.-% $Cr_2O_3$ und 0 – 30 Gew.-% Mn hydriert.

Geeignete aliphatische und cycloaliphatische Kohlenwasserstoffe sind beispielsweise solche mit bis zu 30, vorzugsweise bis zu 20 C-Atomen, z. B. Decan, Dodecan, Tetradecan, Cyclohexan, Cyclooctan, Cyclododecan, Dekalin, Methylcyclohexan.

Geeignete Ether sind beispielsweise Dialkylether wie Diisopropylether, Dibutylether, cyclische Ether wie Dioxan, Tetrahydrofuran, sowie Ether mehrwertiger Alkohole wie Ethylenglykol- und Polyethylenglykol-dimethyl- und -diethylether.

Geeignete Alkohole sind ein- und mehrwertige aliphatische und cycloaliphatische Alkohole wie Methanol, Ethanol, Propanol, Butanol, Cyclohexanol, Ethylenglykol, Propylenglykol, Polyethylenglykol, Ethylenglykol- und Polyethylenglykolmonomethyl- und -monoethylether.

Selbstverständlich können auch Mischungen der genannten Lösungsmittel verwendet werden.

Für die Hydrierung verwendet man ein Reaktionsgemisch, das im allgemeinen etwa 2 bis etwa 100, vorzugsweise etwa 3 bis 30 Teile organisches Lösungsmittel auf 1 Teil Anthrachinon enthält. Dabei ist es nicht unbedingt erforderlich, daß das eingesetzte Anthrachinon bereits zu Beginn der Reaktion vollständig gelöst ist.

Als Träger sind anorganische Materialien wie Kieselgur, Kieselsäuren, Aluminiumoxide, Silikate, Aluminiumsilikate, Montmorillonit, Zeolithe, Bimsstein, Spinelle, Dolomit, Kaolin, Magnesiumsilikate, Zirkonoxid, Titandioxid, Eisenoxid, Zinkoxid, Calciumcarbonat, Siliziumcarbid, Aluminiumphosphat, Borphosphat, Asbest oder Aktiv-Kohle und als organische Katalysatorträger natürlich vorkommende, physikalisch oder chemisch veränderte oder synthetische Verbindungen mit hohem Molgewicht wie Seide, Polyamide, Polystyrole, Zellstoff oder Polyurethane verwendbar, wobei die Träger in Form von Kugeln, Strägen, Fäden, Zylindern, Polygonen oder in Pulverform vorliegen können. Die katalytisch aktiven Elemente können sich homogen im Trägermaterial verteilt oder bevorzugt in der äußeren Schicht oder auf der Oberfläche des Trägers befinden.

Die Katalysatoren können durch Reduktion der Metalloxide hergestellt sein.

Die Reduktion kann mit Wasserstoff, gegebenenfalls bei erhöhter Temperatur und erhöhtem Druck oder unter den Bedingungen des Verfahrens oder während des Verfahrens geschehen.

Die Katalysatoren können als Beschleuniger eines oder mehrere der folgenden Elemente in Mengen bis zu 20 Gew.-% enthalten: Li, Na, Ca, Ba, K, Ag, Be, La, Ce, Nb, Ta, Ti, Mo, W, Zn und bis zu 1 Gew.-% der Elemente Ru, Rh, Pd, Ir, Pt.

Daneben können diese Katalysatoren 0 bis 10 Gew.-% $Al_2O_3$, $SiO_2$, Graphit, Wasserglas oder ein anderes Bindemittel enthalten.

Eine bevorzugte Form der Katalysatoren sind die gegebenenfalls reduzierten Gemische der Oxide des Mn, Cu, Cr, die nur ein Bindemittel in untergeordneter Menge enthalten.

Besonders bevorzugte Katalysatoren enthalten beispielsweise:

a)  50 bis 65 Gew.-% CuO, 35 bis 45 Gew.-% $Cr_2O_3$ und 1 bis 5 Gew.-% Bindemittel,
b)  40 bis 60 Gew.-% CuO, 5 bis 15 Gew.-% BaO, 30 bis 45 Gew.-% $Cr_2O_3$ und 1 bis 5 Gew.-% Bindemittel.

Die katalytische Hydrierung wird im allgemeinen bei Temperaturen zwischen 50 und 250° C, vorzugsweise zwischen 70 und 210° C, durchgeführt. Die Temperatur hängt von der Art des Katalysators und vom Lösungsmittel ab. So erfordern Hydrierungen in weniger polaren Lösungsmitteln höhere Temperaturen.

Die Hydrierung kann bei Wasserstoffdrücken zwischen 1 und 250 bar, vorzugsweise zwischen 5 und 150 bar, durchgeführt werden. Bei einer besonders bevorzugten Verfahrensweise hydriert man bei 10 – 80 bar.

Die Zeit, die für die katalytische Hydrierung benötigt wird, variiert stark, abhängig von den Reaktionsbedingungen. Im allgemeinen ist die

Zeit geringer als 20 Stunden. Die Beendigung der Reaktion kann dadurch erkannt werden, daß das Reaktionsgemisch praktisch keinen Wasserstoff mehr aufnimmt.

Das Verfahren kann diskontinuierlich oder kontinuierlich in den bekannten Hydrierapparaturen wie Hydrierautoklaven, Röhrenreaktoren, Umlaufanlagen oder Kesselkaskaden durchgeführt werden.

Beispielsweise legt man Anthrachinon, Lösungsmittel und Katalysator in einem Hydrierapparat vor und leitet dann bei der gewählten Reaktionstemperatur Wasserstoff unter erhöhtem Druck ein. Das Einleiten des Wasserstoffes kann dabei bis zur Erreichung des gewünschten Wasserstoffdruckes und/oder Ersatz des verbrauchten Wasserstoffes erfolgen. Nach beendeter Reaktion trennt man den Katalysator gegebenenfalls nach Zugabe von weiterem organischem Lösungsmittel ab und isoliert das Anthron, z. B. durch Kristallisation, Abdampfen des Lösungsmittels oder andere bekannte Techniken. Mutterlaugen oder destillierte Lösungsmittel können für den nächsten Ansatz anstelle von neuem Lösungsmittel wieder verwendet werden.

Kontinuierlich führt man das erfindungsgemäße Verfahren beispielsweise in der Weise durch, daß man Anthrachinon als Lösung oder Suspension im organischen Lösungsmittel in einem Röhrenreaktor mit fest angeordnetem körnigen Katalysator oder aber mit aufgewirbeltem, pulverförmigen Katalysator eindosiert und die Hydrierung in der Riesel- oder Sumpfphase durch Einleiten von Wasserstoff im Gleichstrom oder Gegenstrom durchführt. Die Isolierung des Anthrons erfolgt dann wie beschrieben.

Anthron ist ein wichtiges Zwischenprodukt für die Herstellung von Farbstoffen.

### Beispiele

### Beispiel 1

In einem Rührautoklav werden 54 g Anthrachinon in 310 g Cyclohexan in Gegenwart von 3 g eines im Handel erhältlichen Kupferchromit-Katalysators, der 58 Gew.-% CuO, 39 Gew.-% $Cr_2O_3$ und daneben $Al_2O_3$ und ein Bindemittel enthält bei 200°C und einem Wasserstoffdruck von 40 bar hydriert.

Nach 3 Stunden sind bereits 75 Gew.-% Anthron gebildet worden. Die Selektivität der Hydrierung liegt über 99%.

### Beispiel 2

In einem Rührautoklav werden 50 g Anthrachinon in 310 g Cyclohexan zusammen mit 3 g eines Kupfer-Bariumchromit-Katalysators, der 48 Gew.-% CuO, 10 Gew.-% BaO, 37 Gew.-% $Cr_2O_3$ und ein Bindemittel enthält, 3 Stunden bei 200°C und einem Wasserstoffdruck von 40 bar hydriert.

Der Katalysator wird abgetrennt und die Lösung am Rotationsverdampfer eingeengt. Man erhält 43,5 g eines Produktes, das nach gaschromatographischer Analyse nahezu 90 Gew.-% Anthron und weniger als 6 Gew.-% Anthrachinon enthält.

### Beispiel 3

In einem Rührautoklav werden 50 g Anthrachinon in 310 g Cyclohexan in Gegenwart von 5 g eines Katalysators, der als aktive Bestandteile 13 Gew.-% Cu, 13 Gew.-% Mn, 33 Gew.-% $Cr_2O_3$ und 0,8 Gew.-% $Al_2O_3$ enthält, bei 200°C und einem Wasserstoffdruck von 45 bar hydriert. Nach 3 Stunden ist die Umsetzung beendet. Der Katalysator wird abgetrennt und die Lösung am Rotationsverdampfer eingeengt.

Man erhält 43,3 g eines Produktes, das nach gaschromatographischer Analyse ebenfalls nahezu 90 Gew.-% Anthron und weniger als 4 Gew.-% Anthrachinon enthält.

### Beispiel 4

### (Vergleichsbeispiel)

In einem Rührautoklav werden 100 g Anthrachinon in 355 g Dekalin in Gegenwart von 10 g Raney-Ni-Fe (15%ig) 15 Minuten bei 170°C und einem Wasserstoffdruck von 170 bar hydriert. Im Reaktionsgemisch werden gaschromatographisch 11,8 Gew.-% Anthrachinon, 20,0 Gew.-% 1.2.3.4.5.6.7.8-Oktahydro-anthrahydrochinon, 7,6 Gew.-% 1.2.3.4-Tetrahydro-anthrachinon und in der Summe 43,4 Gew.-% 1.2.3.4.5.6.7.8-Oktahydro-anthrachinon und 1.2.3.4.5.6.7.8-Oktahydro-anthranol neben weiteren Produkten bestimmt. Anthron kann nicht nachgewiesen werden.

### Beispiel 5

### (Vergleichsbeispiel)

Verfährt man wie in Beispiel 6, verwendet jedoch als Katalysator Ni auf Kieselgur, so erhält man ein Reaktionsgemisch, das 34,1 Gew.-% 1.2.3.4.5.6.7.8-Oktahydro-anthrahydrochinon und in der Summe 62,6 Gew.-% 1.2.3.4.5.6.7.8-Oktahydro-anthrachinon und 1.2.3.4.5.6.7.8-Oktahydro-anthranol neben geringen Mengen weiterer Produkte enthält.

Ein ähnliches Ergebnis wird auch mit Raney-Nickel bei 100°C erhalten.

**Patentanspruch**

Verfahren zur Herstellung von Anthron durch katalytische Hydrierung von Anthrachinon in einem inerten organischen Lösungsmittel, in Gegenwart eines Katalysators, dadurch gekennzeichnet, daß man gegebenenfalls auf Trägermaterialien aufgebrachte Katalysatoren mit 10 – 60 Gew.-% Cu, 10 bis 50 Gew.-% $Cr_2O_2$ und 0 – 30 Gew.-% Mn verwendet.

## Claim

Process for the preparation of anthrone by catalytic hydrogenation of anthraquinone in an inert organic solvent in the presence of a catalyst, characterised in that optionally supported catalysts are used which contain 10 – 60% by weight of Cu, 10 to 50% by weight of $Cr_2O_3$ and 0 – 30% by weight of Mn.

## Revendication

Procédé de production d'anthrone par hydrogénation catalytique d'anthraquinone dans un solvant organique inerte, en présence d'un catalyseur, caractérisé en ce qu'on utilise des catalyseurs contenant 10 à 60% en poids de Cu, 10 à 50% en poids de $Cr_2O_3$ et 0 à 30% en poids de Mn, éventuellement fixés sur des matières de support.